# EUROPÄISCHE PATENTANMELDUNG

(11) **EP 4 620 510 A1**
(43) Veröffentlichungstag der Anmeldung: **24.09.2025**
(21) Anmeldenummer: 24164107.5
(22) Anmeldetag: 18.03.2024
(51) Int. Cl.: A61M 60/122, H01R 13/52, H01R 13/627, H01R 24/58, A61M 60/20, A61M 60/88

(54) **ANORDNUNG UMFASSEND EINE KOMPONENTE FÜR EINE BLUTPUMPE UND EINEN STECKER**

(71) Anmelder: Berlin Heart GmbH, 12247 Berlin (DE)
(72) Erfinder: Schärff, Eike, 12247 Berlin (DE)
(74) Vertreter: Pfenning, Meinig & Partner mbB

(57) **Zusammenfassung**

Die Offenbarung beschreibt eine Anordnung (1) umfassend eine Komponente (100) für eine Blutpumpe und einen Stecker (200), wobei die Komponente (100) zur Herstellung einer elektrischen Verbindung zur Blutpumpe eine Steckbuchse (102) zur lösbaren Aufnahme des Steckers (200) aufweist. Der Stecker (200) weist dabei einen an einer Spitze (202) des Steckers (200) angeordneten und in die Steckbuchse (102) einführbaren Kopplungsabschnitt (204) auf. Ferner umfasst die Steckbuchse (102) ein elektrisch isolierendes Buchsengehäuse (104), das einen Gehäuseeingang (106) und einen sich an den Gehäuseeingang (106) anschließenden Gehäuseinnenraum (108) zur Aufnahme des Kopplungsabschnitts (204) aufweist, wobei eine Flucht vom Gehäuseeingang (106) in den Gehäuseinnenraum (108) eine Einführungsrichtung (110) des Steckers (200) definiert. Zudem umfasst die Steckbuchse (102) eine Arretiereinheit (112), die ausgebildet ist, den in die Steckbuchse (102) eingeführten Stecker (200) lösbar zu arretieren, wobei die Arretiereinheit ausgebildet ist, den Stecker (200) im Buchsengehäuse (104) zu arretieren ohne dabei eine Rotation des Steckers (200) im Buchsengehäuse (104) um die Einführungsrichtung (110) einzuschränken.

## Beschreibung

Die Erfindung liegt auf dem Gebiet der Medizintechnik, insbesondere der Herzunterstützungssysteme und des elektrischen Apparatebaus.

In der Medizin sind Herzunterstützungssysteme mit Blutpumpen zur Aufrechterhaltung des Blutkreislaufs bekannt. Viele solcher Pumpen, wie beispielsweise VAD- (Ventrical Assist Devices) Pumpen, sind zumindest teilweise implantierbar und auch transportierbar, so dass Personen mit solchen Systemen zumindest teilweise mobil sind.

Für die Steuerung der Blutpumpe und die Versorgung der Blutpumpe mit elektrischer Energie sind die Blutpumpen zumeist mit extrakorporalen Komponenten wie einer Steuereinrichtung verbunden. Die elektrische Verbindung zwischen Blutpumpe und extrakorporaler Komponente erfolgt dabei über ein Verbindungskabel (im englischen *Driveline* genannt). Um die verwendete Komponente, beispielsweise wegen eines Defekts, austauschen zu können, ist das Verbindungskabel mit der Komponente über eine lösbare Steckverbindung elektrisch und mechanisch verbunden. Aufgrund der lebenswichtigen Funktion der Blutpumpe für den Patienten werden an diese Steckverbindung erhöhte Anforderungen gestellt. So muss die Steckverbindung nicht nur eine sichere elektrische Verbindung zwischen Komponente und Blutpumpe ermöglichen. Sie muss außerdem eine mechanische Sicherung umfassen, damit das Verbindungskabel nicht aus Versehen von der Komponente getrennt werden kann. Zudem muss die Steckverbindung ausgelegt sein, wiederholt geschlossen und wieder gelöst zu werden. Gleichzeitig muss gewährleistet sein, dass die Komponente im eingesteckten Zustand gereinigt und desinfiziert werden kann.

Aus dem Stand der Technik sind bereits einige lösbare Verbindungssysteme bekannt.

Die US 2021/0361932 A1 beschreibt beispielsweise Systeme und Verfahren für Verbindungen in einem medizinischen Gerätesystem.

Die US 10,953,145 offenbart eine Verbinderanordnung zum Anschließen externer Stromquellen an ein implantiertes, medizinisches Gerät.

Die Aufgabe, die in dieser Offenbarung adressiert wird, ist, eine verbesserte Verbindungsanordnung zwischen Blutpumpe und einer Komponente für die Blutpumpe bereitzustellen.

Diese Aufgabe wird gelöst durch die in Anspruch 1 beschriebene Anordnung.

Die Anordnung umfasst eine Komponente für eine Blutpumpe und einen Stecker, wobei die Komponente zur Herstellung einer elektrischen Verbindung zur Blutpumpe eine Steckbuchse zur lösbaren Aufnahme des Steckers aufweist. Der Stecker weist einen am distalen Ende des Steckers angeordneten und in die Steckbuchse einführbaren Kopplungsabschnitt auf. Die Steckbuchse weist ihrerseits ein elektrisch isolierendes Buchsengehäuse auf, das einen Gehäuseeingang und einen sich an den Gehäuseeingang anschließenden Gehäuseinnenraum zur Aufnahme des Kopplungsabschnitts umfasst, wobei eine Flucht vom Gehäuseeingang in den Gehäuseinnenraum eine Einführungsrichtung des Steckers definiert. Ferner weist die Steckbuchse auch eine Arretiereinheit auf, die ausgebildet ist, den in die Steckbuchse eingeführten Stecker lösbar zu arretieren, wobei die Arretiereinheit ausgebildet ist, den Stecker im Buchsengehäuse zu arretieren ohne dabei eine Rotation des Steckers im Buchsengehäuse um die Einführungsrichtung einzuschränken.

Stecker und Steckbuchse bilden eine Verbindungsanordnung. Die Arretiereinheit ist wie oben beschrieben ausgebildet, den Stecker innerhalb der Steckbuchse vorzugsweise mechanisch festzuhalten, um ein versehentliches Herausziehen des Steckers aus der Steckbuchse zu vermeiden, und so die Bediensicherheit der Verbindungsanordnung zu erhöhen. Unter der Formulierung, dass die Arretiereinheit ausgebildet ist, den Stecker im Buchsengehäuse zu arretieren ohne dabei eine Rotation des Steckers einzuschränken, wird zum Ausdruck gebracht, dass der Stecker um einen beliebigen Winkelbereich um die Einführungsrichtung des Steckers in der Steckbuchse rotiert werden kann.

Die Erfinder haben erkannt, dass ein bisher ungelöstes medizinisches Problem die Vermeidung von sogenannten Driveline-Infektionen ist. Bei einer Driveline-Infektion handelt es sich um eine Infektion des Gewebes um eine Austrittsstelle der Driveline am Körper des Patienten. Eine Driveline-Infektion wird begünstigt, wenn das Verbindungskabel vielen Bewegungen wie Rotation und Torsion ausgesetzt ist. Durch ihre Arbeit haben die Erfinder erkannt, dass sich Rotationen und Torsionen des Verbindungskabels reduzieren lassen, wenn der Stecker des Verbindungskabels im eingesteckten und verriegelten Zustand beliebig um die Einsteckrichtung rotieren lässt. So kann bei einem Hantieren des Patienten mit der Komponente für die Blutpumpe der Stecker in der Steckbuchse entsprechend einer Relativbewegung der Komponente relativ zum Körper des Patienten mitrotieren, so dass an der Austrittstelle des Verbindungskabels in den Körper des Patienten eine Rotation oder eine Torsion des Kabels reduziert wird. Gleichzeitig bleibt die Bediensicherheit durch die mechanische Arretierung des Steckers innerhalb der Steckbuchse erhalten.

Im Folgenden werden weitere mögliche Ausführungsformen der Anordnung beschrieben.

In einer Ausführungsform kann die Komponente eine Steuereinrichtung sein, die ausgebildet ist, über die Steckbuchse Steuersignale auszugeben und/oder elektrische Energie zum Betreiben der Blutpumpe bereitzustellen. Alternativ kann die Komponente auch eine Energieversorgungseinheit sein, die ausgebildet ist, über die Steckbuchse elektrische Energie zum Betreiben der Blutpumpe bereitzustellen.

In einer anderen Ausführungsform kann der Kupplungsbereich des Steckers rotationssymmetrisch bzgl. der Einführungsrichtung ausgebildet sein. Die rotationsymmetrische Ausbildung stellt eine einfache Designoption dar, die eine Herstellung des Steckers vereinfachen kann. Weiterhin kann so bei einer Rotation des Steckers in der Steckbuchse eine Reibung reduziert werden.

In einer Ausführungsform kann der Kopplungsabschnitt des Steckers einen, vorzugsweise elektrisch isolierten, Arretierabschnitt umfassen. Ferner kann die Arretiereinheit ein Verriegelungselement aufweisen, wobei das Verriegelungselement zwischen einer arretierenden Lage und einer nicht-arretierenden Lage verschiebbar ist und in der arretierenden Lage teilweise in eine Flucht des Gehäuseeingangs hineinragt und ausgebildet ist, bei eingeführtem Stecker in den Arretierabschnitt des Steckers einzugreifen, um einem Herausziehen des Steckers entgegenzuwirken. Es gibt verschiedene Möglichkeiten, die Rotation des Steckers in der Steckbuchse bei gleichzeitiger Arretierung zu gewährleisten. Diese Realisierung der Arretiereinheit kann besonders geeignet sein, um einem versehentlichen Herausziehen des Steckers aus der Steckbuchse entgegenzuwirken.

In einer Variante dieser Ausführungsform kann der Arretierabschnitt zusätzlich einen oder mehrere laterale Vorsprünge aufweisen, die den Kopplungsabschnitt in einer Ebene senkrecht zur Einführungsrichtung zumindest teilweise umgeben und so angeordnet sind, dass diese bei eingeführtem Stecker von dem Verriegelungselement in der arretierenden Lage hintergriffen werden. Die ein oder mehreren Vorsprünge sollten dabei so um den Kopplungsabschnitt herum angeordnet sein, dass die Vorsprünge auch bei einer beliebigen Rotation des Steckers relativ zur Steckbuchse durch das Verriegelungselement hintergriffen werden. Bei dem einen oder mehreren Vorsprüngen kann es sich beispielweise um genau einen Vorsprung handeln, der den Kopplungsabschnitt ringförmig umläuft.

In einer anderen Variante dieser Ausführungsform kann der Arretierabschnitt eine bzgl. der Einführungsrichtung des Steckers den Kopplungsabschnitt zumindest abschnittweise umlaufende Auflagefläche aufweisen, die an der von der Steckerspitze abgewandten Seite des Vorsprungs bzw. der mehreren Vorsprünge angeordnet ist. Dies kann vorteilhaft sein, um eine Reibung des Verriegelungselements im Bereich des Arretierabschnitts zu reduzieren und so eine Rotation des Steckers in der Steckbuchse zu erleichtern. Die Auflagefläche kann sich dabei über den gesamten Umfang des Kopplungsabschnitts erstrecken. Alternativ kann die Auflagefläche auch abschnittweise unterbrochen sein, d.h. beispielsweise ein oder mehrere Ausnehmungen aufweisen. Die Ausnehmungen sollten dabei jedoch so gestaltet sein, dass die Rotierbarkeit des arretierten Steckers in der Steckbuchse erhalten bleibt und beispielsweise das Verriegelungselement weder ganz noch teilweise in die ein oder mehreren Ausnehmungen eingreifen kann.

Auflagefläche und Vorsprung können dabei beispielsweise in einer Variante dieser Ausführungsform als Teil einer den Kopplungsabschnitt bzgl. der Einführungsrichtung umlaufenden Nut ausgebildet sein.

In einer weiteren Variante dieser Ausführungsform kann das Verriegelungselement einen plattenförmigen Abschnitt aufweisen, der quer, vorzugsweise senkrecht, zur Einführungsrichtung angeordnet ist, und einen Riegelrand umfasst, wobei der Riegelrand zum Eingreifen in den Arretierbereich des Steckers einen Abschnitt mit einem kreisbogenförmigen Verlauf aufweist, der in der arretierenden Lage des Verriegelungselements in die Flucht des Gehäuseeingangs hineinragt. Durch den kreisbogenförmigen Verlauf des Riegelrandes kann ein für eine Rotation des Steckers in der Steckerbuchse benötigte Kraft reduziert werden. Der kreisbogenförmige Verlauf des Randes kann dabei vorzugsweise bzgl. des Riegels konkav ausgebildet sein, d.h. eine Verbindungsstrecke zwischen zwei Punkten des kreisbogenförmigen Verlaufs verlaufen nicht durch den Riegel. In einer Realisierung kann der kreisförmige Verlauf einen Bogenabschnitt zusätzlich oder alternativ mindestens 120°, mindestens 160° oder mindestens 180° aufweisen.

In einer anderen Realisierung kann der Riegelrand ein Außenrand des plattenförmigen Abschnitts sein. Der Riegelrand kann dabei beispielsweise durch eine U-förmige Aussparung im plattenförmigen Abschnitt zum Hindurchführen des Steckers gebildet sein. Alternativ hierzu kann der plattenförmige Abschnitt ein Durchgangsloch zum Hindurchführen des Steckers umfassen und der kreisbogenförmige Riegelrand durch einen Rand des Durchgangslochs gebildet sein. Das Durchgangsloch kann dabei oval ausgebildet sein und der Riegelrand zwei halbkreisförmige Abschnitte mit gleichem Radius, die durch zwei gradlinige Abschnitte miteinander verbunden sind, aufweisen.

In einer anderen Realisierung des Verriegelungselementes mit plattenförmigen Abschnitt kann der plattenförmige Abschnitt eine Stirnfläche umfassen, die in Richtung des Gehäuseeingangs ausgerichtet ist, und ein Übergang zwischen Stirnfläche und Riegelrand kann eine Fase aufweisen. Durch die Fase kann das Einführen des Steckers in die Steckbuchse erleichtert werden, indem die Fase ein temporäres Verschieben des Verriegelungselements aus der arretierenden Lage in die nicht-arretierende Lage durch das Einführen des Steckers ermöglicht oder vereinfacht.

In einer anderen Variante der Ausführungsform kann die Arretiereinheit zusätzlich oder alternativ eine Rückstelleinheit umfassen, die ausgebildet ist, wenn keine äußere Krafteinwirkung vorliegt, das Verriegelungselement in der arretierenden Lage zu fixieren.

In einer weiteren Ausführungsform kann die Komponente ein Betätigungselement aufweisen, das mit der Arretiereinheit verbunden ist, und die Arretiereinheit ausgebildet sein, bei einem Betätigen des Betätigungselementes die Arretierung aufzuheben. In einer Variante dieser Ausführungsform, die ebenfalls die Rückstelleinheit aufweist, kann die Arretiereinheit ausgebildet sein, bei einem Betätigen des Betätigungselementes einer durch die Rückstelleinheit erzeugten Kraft entgegenzuwirken, um den Riegel in die nicht-arretierende Lage zu bewegen. Dies kann vorteilhaft sein, um einem Patienten oder einem anderen Benutzer der Komponente eine komfortable Möglichkeit zu geben, um die Arretierung des Steckers in der Steckbuchse aufzuheben.

In einer anderen Ausführungsform kann bzw. können im Kopplungsabschnitt und innerhalb des Gehäuseinnenraums jeweils ein oder mehrere elektrische Kontaktelemente zur Herstellung einer elektrischen Verbindung zwischen der Komponente und dem Stecker angeordnet sein.

In einer Variante dieser Ausführungsform können die ein oder mehreren elektrischen Kontakte innerhalb der Steckbuchse zwischen Gehäuseeingang und Arretiereinheit oder auf einer vom Gehäuseeingang abgewandten Seite der Arretiereinheit angeordnet sein. Eine Anordnung von Arretierabschnitt und elektrischen Kontakten entlang des Kopplungsabschnitts des Steckers kann dazu entsprechend realisiert sein.

In einer anderen Variante dieser Ausführungsform können die elektrischen Kontaktelemente des Steckers auf einer Außenseite des Kopplungsabschnitts und die Kontaktelemente der Steckbuchse in einem, vorzugsweise teilweise zylindrischen Abschnitt des Gehäuseinnenraums angeordnet sein. Alternativ hierzu kann der Kopplungsabschnitt des Steckers in Form eines Hohlzylinders mit einer Hohlzylinderöffnung in Einführungsrichtung ausgebildet sein und die Kontaktelemente des Steckers können innerhalb des Hohlzylinders angeordnet sein. Die Steckbuchse umfasst in diesem Fall einen Stift, an dessen Oberfläche die Kontaktelemente der Steckbuchse angeordnet sind.

In einer anderen Variante können die Kontaktelemente hintereinander entlang der Einführungsrichtung auf bzw. im Stecker und in der Steckbuchse angeordnet sein. Zudem können die Kontaktelemente entweder bei dem Stecker als Kontaktflächen und bei der Steckbuchse als federgelagerte Kontaktabnehmer ausgebildet sein oder anders herum. Federgelagerte Kontaktabnehmer können bspw. Ringfederkontakte oder Stiftfederkontakte sein.

In einer Variante dieser Ausführungsform kann der Stecker im Kopplungsabschnitt ein Steckerdichtelement umfassen und zwischen Gehäuseeingang und den ein oder mehreren elektrischen Kontaktelementen der Steckbuchse ein Steckbuchsendichtelement angeordnet sein, das ausgebildet ist, bei eingeführtem Stecker mit dem Steckerdichtelement zusammenzuwirken, um einem Eindringen von Flüssigkeit und/oder Partikeln in den Gehäuseinnenraum entgegenzuwirken. Das Dichtelement des Steckers kann zusätzlich ferner beispielsweise als Ringdichtung ausgebildet sein und das Dichtelement der Steckbuchse als Dichtungsauflagefläche oder anders herum.

In einer Realisierung dieser Variante kann das Dichtelement der Steckbuchse zwischen Gehäuseeingang und Arretiereinheit angeordnet sein. Dies kann dazu beitragen, dass nicht nur die elektrischen Kontaktelemente sondern auch die Arretiereinheit vor Partikeln und Flüssigkeit geschützt wird.

In einer weiteren Ausführungsform kann der Gehäuseinnenraum, um ein Einführen des Steckers in das Buchsengehäuse zu begrenzen, eine Anschlagfläche umfassen, die in Richtung des Gehäuseeingangs ausgerichtet ist, und der Stecker mindestens einen lateralen Vorsprung umfassen, der eine in Richtung der Spitze des Steckers ausgerichtete Stirnfläche aufweist, die bei eingeführtem Stecker an der Anschlagsfläche anliegt. Die Anschlagsfläche kann dabei beispielsweise quer zur Einführungsrichtung ausgerichtet sein.

In einer Variante dieser Ausführungsform kann beispielsweise die Anschlagsfläche durch eine Grenzfläche zwischen zwei aneinander angrenzende Abschnitte des Gehäuseinnenraums mit unterschiedlichen Durchmessern realisiert sein, wobei der Abschnitt mit dem größeren Durchmesser näher am Gehäuseeingang angeordnet ist. Der Vorsprung des Steckers kann der gleiche Vorsprung sein, wie der Vorsprung des Arretierbereichs.

Alternativ oder zusätzlich kann zur Anschlagsfläche der Stecker auch eine optisch sichtbare Markierung aufweisen, anhand derer ablesbar ist, ob der Stecker ausreichend weit in die Steckbuchse eingeführt ist.

In einer Variante dieser Ausführungsform kann die Stirnfläche des Vorsprungs eine Fase aufweisen, die die Stirnfläche lateral zumindest teilweise umgibt. Diese kann ein Einführen des Steckers in die Steckbuchse vereinfachen.

In einer anderen Ausführungsform der Anordnung kann die Anordnung eine Blutpumpe und ein Verbindungskabel, das den Stecker umfasst und mit dem die Blutpumpe mit der Komponente elektrisch verbindbar ist, aufweisen.

Im Folgenden werden nun konkrete Ausführungsbeispiele der Anordnung aus Komponente und Stecker anhand der Figuren beschrieben. Dazu wird zunächst ein Überblick über das in den Figuren gezeigte gegeben.
- Fig. 1: zeigt eine Anordnung aus einer Komponente mit einer Steckbuchse und einem Verbindungskabel mit einem Stecker zur Herstellung einer elektrischen Verbindung zwischen der Komponente und der Blutpumpe;
- Fig. 2: zeigt den Stecker und einen Querschnitt durch die Steckbuchse aus Fig. 1 in einer Vergrößerung;
- Fig. 3: zeigt einen Querschnitt der Steckbuchse aus Fig. 1 entlang einer Einführungsrichtung in einer Vergrößerung;
- Fig. 4: zeigt eine Ausführungsform des Verriegelungselements, wie es in der Steckbuchse aus Fig. 1 verwendet wird;
- Fig. 5: zeigt den Stecker aus Fig. 1 einzeln in einer Vergrößerung;
- Fign. 6-8: zeigen den Stecker und die Steckbuchse aus Fig. 1 in verschiedenen Phasen während eines Einführens und Entnehmens des Steckers in bzw. aus der Steckbuchse;
- Fig. 9: zeigt eine zur Fig. 1 alternative Ausführungsform eines Steckers und einer Steckbuchse;
- Fig. 10: zeigt ein Verriegelungselement, wie es in der alternativen Ausführungsform der Steckbuchse aus Fig. 9 verwendet wird;
- Fig. 11: zeigt den Stecker aus Fig. 1 mit einer zur Fig. 1 alternativen Ausführungsform einer Steckbuchse;
- Fig. 12: zeigt die Anordnung 1 in Verbindung mit einer Blutpumpe.

Im Folgenden werden nun die Ausführungsbeispiele aus den Figuren im Detail beschrieben. In der nachfolgenden Beschreibung und in den Figuren sind identische Merkmale mit dem gleichen Bezugszeichen versehen. Aus Übersichtlichkeitsgründen sind Bezugszeichen teilweise nicht in jedem Beispiel angegeben, auch wenn das zugehörige Merkmal in der betreffenden Figur gezeigt ist.

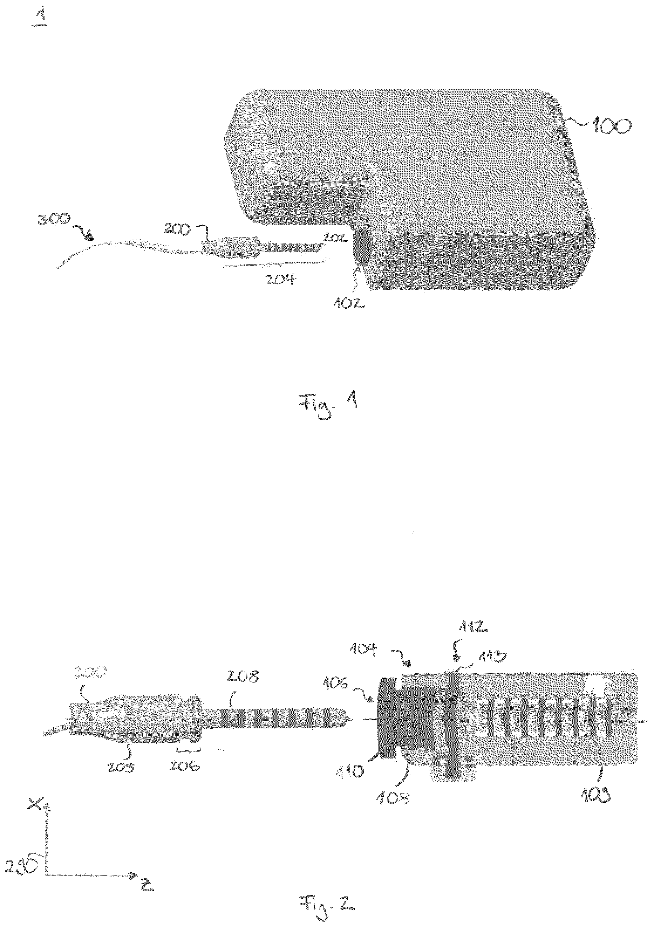

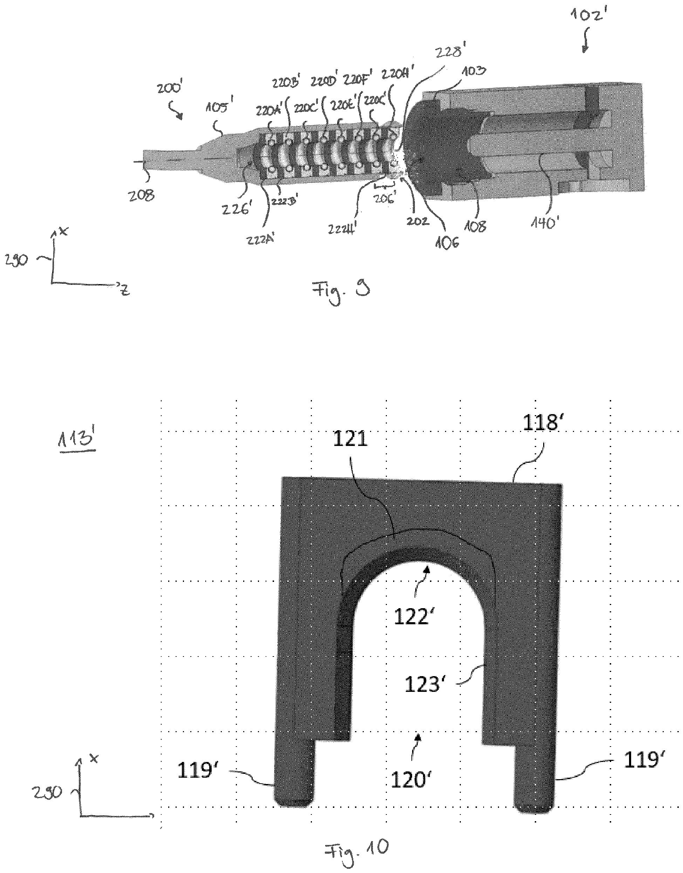

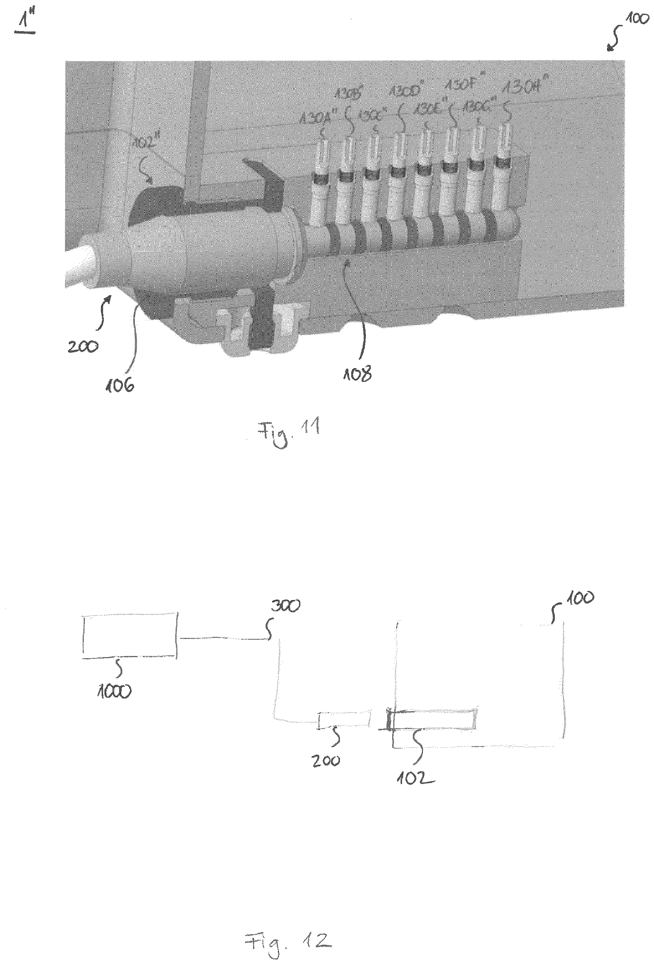

Zunächst wird ein erstes Ausführungsbeispiel der Anordnung anhand der Figuren 1 bis 8 beschrieben. Im Anschluss werden zwei alternative Ausführungsbeispiele anhand der Fign. 9 und 10 sowie anhand der Fig. 11 beschrieben. Abschließend wird die Anordnung aus Fign. 1 bis 8 mit Bezug auf Fig. 12 in Verbindung mit einer Blutpumpe beschrieben. Im Hinblick auf das erste Ausführungsbeispiel wird anhand von Fign. 1 und 2 zunächst ein Überblick über das erste Ausführungsbeispiel der Anordnung gegeben.

Fig. 1 zeigt eine Anordnung 1, die eine Komponente 100 mit einer Steckbuchse 102 und ein Verbindungskabel 300 mit einem Stecker 200 zur Herstellung einer elektrischen Verbindung zwischen der Komponente 100 und einer Blutpumpe umfasst. Fig. 2 zeigt den Stecker 200 und einen Querschnitt durch die Steckbuchse 102 aus Fig. 1 in einer Vergrößerung.

Die Komponente 100 ist in dem gezeigten Ausführungsbeispiel eine Steuereinrichtung für die Blutpumpe, die ausgebildet ist, eine Blutpumpe über die Steckbuchse 102 und das Verbindungskabel 300 mit dem Stecker 200 mit elektrischer Energie zu versorgen. Gleichzeitig sind über in den Figuren nicht gezeigte Eingabeschnittstellen der Steuereinrichtung 100 Betriebsparameter für einen Betrieb der Blutpumpe wählbar. Ferner ist die Steuereinrichtung 100 ausgebildet, entsprechend der gewählten Betriebsparameter Steuersignale über die Steckbuchse 102 an die Blutpumpe zu übermitteln.

Der Stecker 200 umfasst einen an seinem distalen Ende bzw. der Spitze 202 des Steckers 200 angeordneten und in die Steckbuchse 102 einführbaren Kopplungsabschnitt 204. Über den Kopplungsabschnitt 204 ist eine mechanische wie auch eine elektrisch leitende Verbindung zwischen dem Stecker 200 und der Steckbuchse 102 herstellbar. Die Steckbuchse 102 ist ausgebildet, den Stecker 200 lösbar aufzunehmen und weist hierzu ein elektrisch isolierendes Buchsengehäuse 104 auf, das einen Gehäuseeingang 106 und einen sich an den Gehäuseeingang 106 anschließenden Gehäuseinnenraum 108 zur Aufnahme des Kopplungsabschnitts 204 umfasst. Eine Flucht des Gehäuseeingangs 106 in den Gehäuseinnenraum 108 definiert dabei eine Einführungsrichtung 110 des Steckers 200 in die Steckbuchse 102. Die Einführungsrichtung 110 fällt dabei für die Steckbuchse 102 mit einer Längsachse 109 des Gehäuseinnenraums 108 zusammen und für den Stecker 200 mit einer Längsachse 208 des Steckers 200. Zur besseren Beschreibung von Ausdehnungen und Anordnungen von Bestandteilen der Anordnung 1 über die einzelnen Figuren hinweg ist in Fig. 2 ebenfalls ein Koordinatensystem 290 eingezeichnet, dessen z-Achse parallel zur Einführungsrichtung 110 verläuft.

Die Steckbuchse 102 umfasst weiterhin eine Arretiereinheit 112, die ausgebildet ist, den in die Steckbuchse 102 eingeführten Stecker 200 lösbar zu arretieren. Die Arretiereinheit 112 ist dabei ausgebildet, den Stecker 200 im Buchsengehäuse 104 zu arretieren ohne dabei eine Rotation des Steckers 200 im Buchsengehäuse 104 um die Einführungsrichtung 110 einzuschränken.

Die Formulierung, dass die Rotation des Steckers 200 in der Steckbuchse 102 um die Einführungsrichtung 110 nicht eingeschränkt wird, bedeutet, dass die Rotation des Steckers 200 innerhalb der Steckbuchse 102 nicht durch einen Endanschlag behindert wird. In dem gezeigten Ausführungsbeispiel ist die Rotation des Steckers 200 um einen beliebigen Winkelbereich möglich. Unter den Begriff der Einschränkung ist dabei jedoch nicht zu verstehen, dass bei einer Rotation zwischen Stecker 200 und Steckbuchse 102 keine Reibung auftritt. Diese ist auch im gezeigten Ausführungsbeispiel vorhanden. Die Reibung schränkt jedoch die Rotation des Steckers 200 prinzipiell nicht ein, sondern führt lediglich zu einem höheren Kraftaufwand für die Rotation des Steckers 200 in der Steckbuchse 102.

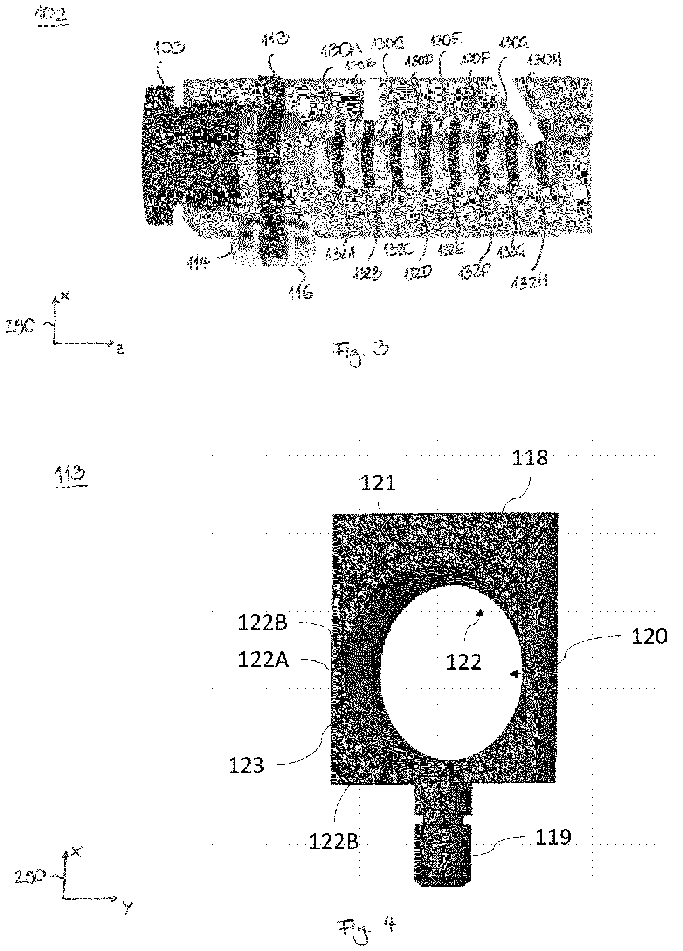

Es gibt verschiedene Möglichkeiten, die Arretiereinheit 112 und den Stecker 200 auszubilden, um eine Rotation des Steckers 200 im Buchsengehäuse 104 zu gewährleisten. In dem in den Fign. 1 bis 8 gezeigten Ausführungsbeispiel der Anordnung 1 wird dies dadurch erreicht, dass der Kopplungsabschnitt 204 des Steckers 200 einen elektrisch isolierten Arretierabschnitt 206 umfasst und die Arretiereinheit 112 ein Verriegelungselement 113 aufweist, wobei das Verriegelungselement 113 zwischen einer arretierenden Lage und einer nicht-arretierenden Lage verschiebbar ist und in der arretierenden Lage teilweise in die Flucht des Gehäuseeingangs 106 hineinragt und ausgebildet ist, bei eingeführtem Stecker 200 in den Arretierabschnitt 206 des Steckers 200 einzugreifen, um einem Herausziehen des Steckers 200 entgegenzuwirken. Details des Arretierabschnitts 206 des Steckers 200 und der Arretiereinheit 112 werden im Folgenden anhand der Fign. 3 bis 8 beschrieben. Zunächst wird dabei auf Fign. 3 bis 5 eingegangen.

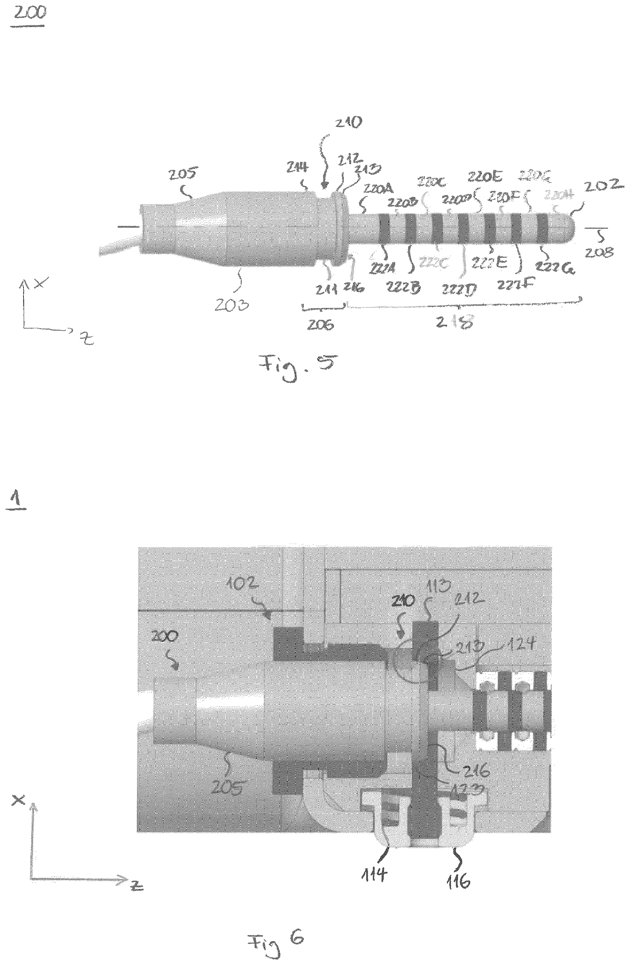

Fig. 3 zeigt einen Querschnitt der Steckbuchse 102 aus Fig. 1 entlang der Einführungsrichtung 110 in einer Vergrößerung. Fig. 4 zeigt ein Ausführungsbeispiel des Verriegelungselements 113, wie es in der Steckbuchse 102 aus Fig. 1 verwendet wird. Fig. 5 zeigt den Stecker 200 aus Fig. 1 einzeln in einer Vergröβerung.

Zunächst wird mit Bezug auf Fign. 3 und 4 die Arretiereinheit 112 der Steckbuchse 102 näher erläutert. Die Arretiereinheit 112 weist ein Verriegelungselement 113 auf. Das Verriegelungselement 113 ist zwischen einer arretierenden Lage und einer nicht-arretierenden Lage verschiebbar, wobei das Verriegelungselement 113 in der arretierenden Lage teilweise in die Flucht des Gehäuseeingangs 106 hineinragt und ausgebildet ist, bei eingeführtem Stecker 200 in den Arretierabschnitt 206 des Steckers 200 einzugreifen, um einem Herausziehen des Steckers 200 entgegenzuwirken. In der arretierenden Lage ragt dabei ein oberer Bereich des Verriegelungselements 113 in die Flucht des Gehäuseeingangs 106 rein. Um von der arretierenden Lage in die nicht-arretierende Lage gebracht zu werden, muss das Verriegelungselement 113 in positive x-Richtung verschoben werden. In der nicht-arretierenden Lage ragt dann aufgrund eines Durchgangslochs kein Anteil des Verriegelungselements 113 in die Flucht des Gehäuseeingangs 106 hinein.

In dem in Fign. 1 bis 8 gezeigten Ausführungsbeispiel verfügt die Arretiereinheit 112 weiterhin über eine Feder 114 als Rückstelleinheit, die ausgebildet ist, wenn keine äußere Krafteinwirkung vorliegt, das Verriegelungselement 113 in der arretierenden Lage zu fixieren. Weiterhin verfügt die Komponente über ein Betätigungselement 116, das mit der Arretiereinheit 113 verbunden ist. Ferner ist die Arretiereinheit 113 ausgebildet, bei einem Betätigen des Betätigungselementes 116 die Arretierung aufzuheben. Bei dem gezeigten Ausführungsbeispiel, ist die Feder 114 so mit dem Verriegelungselement 113 verbunden, dass die Feder permanent mit einer Rückstellkraft in die negative x-Richtung wirkt, so dass das Verriegelungselement 113 ohne äußere Krafteinwirkung in der arretierenden Lage gehalten wird bzw. nach einem Verschieben des Verriegelungselements 113 in die nicht-arretierende Lage durch die Feder 114 wieder in die arretierende Lage zurückgebracht wird. Ferner ist die Feder 114 innerhalb des Betätigungselements 116 angeordnet. In diesem Ausführungsbeispiel handelt es sich bei dem Betätigungselement 116 um einen mechanischen Knopf. Durch eine Krafteinwirkung in positive x-Richtung auf das Betätigungselement 116, wird die die Feder 114 zusammengedrückt und gleichzeitig das Verriegelungselement 113 in positive x-Richtung aus der arretierenden Lage in die nicht-arretierende Lage verschoben. Dies ermöglicht ein Lösen des Steckers 200 aus der Steckbuchse 102. In diesem Ausführungsbeispiel hat die Feder 114 dabei eine Doppelfunktion: Das Rückstellen des Verriegelungselements 113 in die arretierende Lage und das Rückstellen des Betätigungselements 116 in eine Ausgangslage. Die gezeigte Anordnung von Verriegelungselement, Rückstelleinheit und Betätigungselement ist jedoch nur ein Beispiel. Das Betätigungselement könnte bspw. anstatt rein mechanisch mit einem elektrischen Anteil realisiert werden, so dass, beispielsweise das Betätigungselement nicht in mechanischer Verbindung zu dem Rückstelleinheit und dem Verriegelungselement steht.

In Fig. 4 ist das Verriegelungselement 113 einzeln in Vorderansicht dargestellt, d.h. betrachtet mit Blickrichtung entlang der positiven z-Achse. Das Verriegelungselement 113 in dem Ausführungsbeispiel der Anordnung 1 weist einen plattenförmigen Abschnitt 118 auf, der, wenn das Verriegelungselement 113 in die Steckbuchse 102 eingebaut ist, senkrecht zur Einführungsrichtung angeordnet ist, d.h. in x-y-Ebene. Das Verriegelungselement 113 umfasst weiterhin einen Stift 119, zur mechanischen Kraftübertragung zwischen dem Betätigungselement 116 und dem Verriegelungselement 113. Ferner umfasst das Verriegelungselement 113 ein Durchgangsloch 120, dessen Randbereich einen Riegelrand 122 bildet. Der Riegelrand 122 wird aus zwei sich gegenüberliegenden halbkreisförmigen Abschnitten 122B mit gleichem Radius gebildet, die durch zwei sich ebenfalls gegenüberliegenden gradlinigen Abschnitten 122A miteinander verbunden sind. Das Durchgangsloch 120 dient zum abschnittweisen Hindurchführen des Kopplungsbereichs 204 des Steckers 200. Ein oberer Bereich 121 des kreisbogenförmigen Riegelrands 122 ragt in der arretierenden Lage des Verriegelungselements 113 in die Flucht der Gehäuseeingangs 106 hinein und dient dem Eingreifen der Arretiereinheit 112 in den Arretierbereich 206 des Steckers 200. Dies wird im Folgenden näher mit Bezug auf Fig. 5 erläutert.

Fig. 5 zeigt eine Vergrößerung des Steckers 200. Wie in Fig. 5 zu sehen ist, ist der Arretierabschnitt 206 in einem Griffelement 205 des Steckers 200 angeordnet. Das Griffelement 205 kann aus einem elektrisch isolierenden Material hergestellt sein. In dem gezeigten Ausführungsbeispiel ist das Griffelement 205 aus einem Polymer hergestellt. Der Arretierabschnitt 206 ist in einem zur Steckerspitze 202 zeigenden Ende des Griffelements 205 angeordnet und umfasst eine in das Griffelement 205 eingearbeitete Nut 210, die den Stecker 200 symmetrisch zur Längsachse 208 vollumfänglich umläuft. Seitenkanten der Nut 210 bilden dabei zwei laterale Vorsprünge 212 und 214, die den Kopplungsabschnitt 204 in einer Ebene senkrecht zur Einführungsrichtung 110 umlaufen. Die Nut 210 ist dabei so angeordnet, dass der Bereich 121 des Riegelrands 122 des Verriegelungselements 113 in der arretierenden Lage in die Nut 210 eingreift und dabei den Vorsprung 212 hintergreift. Eine in der Vertiefung der Nut geformte Oberfläche 211 bildet weiterhin eine Auflagefläche für den Riegelrand 122. Der gesamte Stecker 200 ist in diesem Beispiel rotationssymmetrisch bzgl. der Einführungsrichtung 110 ausgebildet. Dies ist jedoch keine zwingende Voraussetzung. Zum Beispiel kann statt des umfänglich angeordneten lateralen Vorsprungs 212 auch eine Mehrzahl von voneinander beabstandeten, lateralen Vorsprüngen umfänglich angeordnet sein. Die einzige Voraussetzung hierbei ist, dass trotz der Beabstandung der Vorsprünge auch bei einer Rotation des Steckers 200 in der Steckbuchse 102 ein ununterbrochenes Hintergreifen von mindestens einem Vorsprung durch das Verriegelungselement gewährleistet ist.

Im Folgenden wird das Zusammenspielen von Stecker 200 und Steckbuchse 102 während des Einführens des Steckers 200 in die Steckbuchse 102 mit Bezug auf die Fign. 6 bis 8 detaillierter erläutert.

Fign. 6 bis 8 zeigen den Stecker 200 und die Steckbuchse 102 aus Fign. 1 bis 5 in verschiedenen Phasen während eines Einführens und Entnehmens des Steckers 200 in bzw. aus der Steckbuchse 102.

In Fig. 6 ist eine Phase des Einführens des Steckers 200 in die Steckbuchse 102 dargestellt. In der in Fig. 1 dargestellten Phase wird der Vorsprung 212 des Steckers 200 gerade durch die Öffnung 120 des Verriegelungselements 113 geschoben. Hierbei ist zu erkennen, wie die Fase 213 an der Stirnseite 216 des Griffelementes 205 des Steckers 200 mit der Fase 123 des Verriegelungselements 113 zusammenwirkt, um das Verriegelungselement 113 bei Einführen des Steckers 200 in z-Richtung von der arretierenden Lage in die nicht-arretierende Lage zu verschieben, damit der laterale Vorsprung 212 das Durchgangsloch 120 des Verriegelungselements 113 passieren kann.

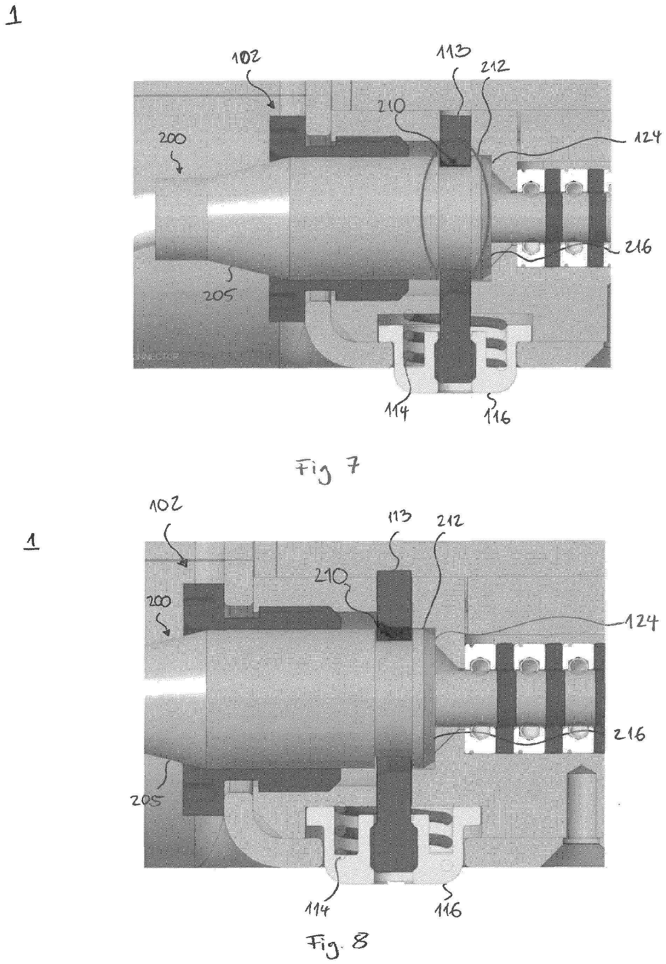

Sobald der Vorsprung 112 das Durchgangsloch 120 passiert hat, wird das Verriegelungselement 113 aufgrund der Rückstellkraft der Feder 114 wieder in die arretierende Lage zurückgeführt, bei der der obere Bereich 121 des Riegelrands 122 in die Nut 210 eingreift. Dies ist in Fig. 7 gezeigt. Das Hintergreifen des Vorsprungs 212 durch den oberen Bereich 121 des Riegelrandes 122 wirkt dabei einem Herausziehen des Steckers 200 aus der Steckbuchse 102 entgegen. Der Stecker 200 kann nicht aus Versehen aus der Steckbuchse 102 gezogen werden. Ebenfalls ist in Fig. 6 dargestellt, wie die Stirnseite 216 des Steckers 200 mit einer Anschlagfläche 124 der Steckbuchse zusammenwirkt, um das Einführen des Steckers 200 in die Steckbuchse 102 in Einführungsrichtung 110 zu begrenzen. Die Anschlagfläche 124 ist dabei eine Grenzfläche zwischen zwei aneinander angrenzende, etwa zylindrisch ausgebildete Bereiche des Gehäuseinnenraums 108 mit unterschiedlichen Radien.

Der laterale Vorsprung 212 hat damit eine Doppelfunktion. Zum einen wirkt der Vorsprung 212 zusammen mit dem Verriegelungselement 113 einem versehentlichen Herausziehen des Steckers 200 aus der Steckbuchse 102 entgegen. Zum anderen begrenzt die Stirnseite 216 des lateralen Vorsprungs 212 das Einführen des Steckers 200 in die Steckbuchse 102, so dass das Einführen des Steckers 200 nicht zu einer Beschädigung des Gehäuseinnenraums 108 führen kann. Zusätzlich sind Stirnseite 216 und Anschlagsfläche 124 so relativ zu Verriegelungselement 113 und Nut 210 angeordnet, dass bei einem vollständig eingeführten Stecker 200 das Verriegelungselement 113 in die Nut 210 des Steckers 200 eingreifen kann.

Abschließend ist in Fig. 8 noch eine erste Phase beim Herausziehen des Steckers 200 aus der Steckbuchse 102 dargestellt. Soll der Stecker 200 aus der Steckbuchse 102 herausgezogen werden, muss zunächst das Verriegelungselement 113 der Steckbuchse 102 aus der arretierenden Lage in die nicht arretierende Lage gebracht werden. Dies kann durch einen Druck auf das Betätigungselement 116 geschehen. Der Druck muss dabei so stark sein, dass dieser die Rückstellkraft der Feder 114 überwindet und das Verriegelungselement 113 in z-Richtung so weit verschoben wird, dass der Riegelrand 122 nicht mehr in die Nut 210 des Steckers 200 eingreift. Diese Phase ist in Fig. 8 dargestellt. Der Stecker 200 kann hieran anschließend durch eine Bewegung entgegengesetzt zur Einführungsrichtung 110 aus der Steckbuchse 102 entfernt werden.

Nachdem die Funktionsweise der Arretiereinheit 112 im Detail beschrieben wurde, wird im Folgenden die Herstellung der elektrischen Verbindung zwischen Stecker 200 und Steckbuchse 102 beschrieben. Hierzu wird noch einmal Bezug auf die Fign. 3 und 5 genommen.

In Fig. 3 ist zu sehen, dass die Steckbuchse 102 insgesamt 8 Kontaktelemente 130A-H aufweist, die als Ringfederkontakte ausgebildet und entlang der Einführungsrichtung hintereinander in einem hinteren Bereich des Gehäuseinnenraums 108 angeordnet sind. Die Ringfederkontakte 130A-H sind dabei durch elektrisch isolierende Ringelemente 132A-H voneinander elektrisch isoliert, wobei zwischen zwei nebeneinander angeordneten Ringfederkontakten immer ein isolierendes Ringelement angeordnet ist. In Fig. 5 ist dargestellt, dass in einem an der Steckerspitze 202 befindlichen Kontaktabschnitt 218 des Kopplungsabschnitts 204 eine Anzahl von Kontaktflächen 220A-H entlang der Einführungsrichtung 110 des Steckers 200 angeordnet sind, die durch elektrisch isolierende Ringelemente 222A-G voneinander elektrisch isoliert sind. Die Kontaktflächen 220A-H sind dabei entlang des Steckers 200 so angeordnet, dass beim vollständigen Einführen des Steckers eine elektrische Verbindung zwischen je einem der Ringfederkontakte 130A-H mit einer der Kontaktflächen 220A-H realisiert wird. In dem Beispiel sind jeweils 8 Kontaktelemente vorhanden. Die Zahl der Kontaktelemente kann jedoch je nach Anforderung frei variiert werden. In dem gezeigten Beispiel ist die Arretiereinheit 112 in der Steckbuchse 102 zwischen Gehäuseeingang 106 und den Ringkontakten 130A-H angeordnet. Wie später auch anhand eines anderen Beispiels gezeigt werden wird, ist diese Anordnung jedoch nicht zwingend notwendig.

Abschließend wird im Folgenden anhand von Fign. 3, 5 und 7 noch beschrieben, wie die Verwendung von Dichtelementen ein Eindringen von Flüssigkeiten und Schmutzpartikeln entgegenwirken kann. In der Steckbuchse 102 ist dafür eine Dichtlippe 103 vorgesehen, die auch den Gehäuseeingang 106 bildet. Der Stecker 200 umfasst im Gegenzug eine Dichtoberfläche 203, die sich an einer von der Steckerspitze 208 abgewandten Seite des Griffelements 205 befindet. Die Dichtlippe 103 der Steckbuchse 102 ist aus einem weichen Kunststoff geformt und liegt, wie in Fig. 7 gezeigt ist, bei einem eingeführten Stecker 200 auf der Dichtoberfläche 203 des Steckers 200 auf. Dichtlippe 103 und Dichtoberfläche 203 wirken dabei zusammen, um ein Eindringen von Schmutz und Flüssigkeit in den Gehäuseinnenraum 108 zu minimieren. In anderen Ausführungsbeispielen ist es jedoch auch möglich statt der Dichtlippe 103 eine Ringdichtung zu verwenden. Zusätzlich oder alternativ kann auch Dichtlippe und Dichtoberfläche an Stecker und Steckbuchse vertauscht angeordnet werden.

Im letzten Teil dieser Offenbarung werden anhand der Fign. 9 bis 11 noch weitere alternative Ausführungsbeispiele der Komponente und des Steckers beschrieben. Zunächst wird dazu eine erste alternative Ausbildung anhand der Fign. 9 und 10 beschrieben.

Fig. 9 zeigt eine zur Fig. 1 alternative Ausführungsform eines Steckers 200' und einer Steckbuchse 102' für eine Komponente. Stecker 200' wie auch Steckbuchse 102' werden in Fig. 9 in einem Querschnitt entlang der Einführungsrichtung 110 von Stecker 200' in die Steckbuchse 102' gezeigt. Fig. 10 zeigt ein Verriegelungselement 113', wie es in der alternativen Ausführungsform der Steckbuchse 102' aus Fig. 9 verwendet wird.

Der Stecker 200' umfasst ein Griffelement 105', das länglich ausgebildet ist und in z-Richtung, d.h. entlang seiner Längsachse des Steckers 200', in die Steckbuchse 102' einführbar ist. Im Unterschied zum Stecker 200 ist das Griffelement 105' jedoch entlang der Längsachse 208 hohlzylindrisch ausgebildet und umfasst eine Hohlraumöffnung 228', an die sich ein Hohlraum 226' anschließt. Innerhalb dieses Hohlraums sind insgesamt 8 als Ringfedern ausgebildete Kontaktelemente 220A'-H' angeordnet, wobei jeweils zwei benachbarte Ringfedern 220A'-H' durch einen elektrisch isolierenden Ring 222A'-H' voneinander getrennt werden. Weiterhin umfasst der Stecker 200' auch einen Arretierabschnitt 206'. Dieser ist jedoch nicht an einem von der Steckerspitze 202 abgewandten Ende des Griffelements 205' angeordnet, wie bei Stecker 200, sondern befindet sich in unmittelbarer Nähe zur Steckerspitze 202.

Die Steckbuchse 102', wie die Steckbuchse 102, umfasst den Gehäuseeingang 106, an den sich ein Gehäuseinnenraum 108 anschließt. Der Gehäuseeingang 106 wird dabei wie auch bei Steckbuchse 102 durch das Dichtelement 103 gebildet. Weiterhin umfasst die Steckbuchse 102' auch eine Arretiereinheit, von der in Fig. 8 jedoch nur das Verriegelungselement 113' gezeigt ist. Im Unterschied zur Steckbuchse 102 ist das Verriegelungselement 113' bei der Steckbuchse 102'an einem von der Gehäuseeingang 106 abgewandten Ende des Gehäuseinnenraums 108 angeordnet. Für die Anordnung der zum Stecker 200' kompatiblen Kontaktelemente ist innerhalb des Gehäuseinnenraums 108 ein Stift 140' angeordnet, der in Fig. 8 im Querschnitt sichtbar ist. Auf einer vom Betrachter abgewandten Seite des Stiftes 140', und deshalb in Fig. 8 nicht zu sehen, sind 8 Kontaktelemente in Form von Kontaktflächen angeordnet, die bei eingeführtem Stecker 200' mit den Ringelementen 220A'-H' eine elektrisch leitende Verbindung zwischen Stecker 200' und Steckbuchse 102' herstellen. Im Folgenden wird nun noch das Verriegelungselement 113' anhand von Fig. 10 näher beschrieben.

Fig. 10 zeigt das Verriegelungselement 113' in der x-y-Ebene, d.h. in einer Ebene senkrecht zur Einführungsrichtung 110. Ferner ist das Verriegelungselement 113' aus der Perspektive eines einzuführenden Steckers 200' abgebildet, d.h. mit Blickrichtung in die positive z-Achse.

Wie das Verriegelungselement 113 umfasst auch das Verriegelungselement 113' einen plattenförmigen Abschnitt 118', der jedoch kein Durchgangsloch umfasst, sondern eine U-förmige Ausnehmung 120'. Die U-förmige Ausnehmung 120' ist dabei so innerhalb des Verriegelungselements 113' eingebracht, dass diese ebenfalls den oberen Bereich 121 aufweist, der als ein kreisbogenförmiger Randbereich 122' der Ausnehmung 120' ausgebildet ist und so angeordnet ist, dass dieser bei einem eingeführten Stecker 200' in die Nut 110 des Steckers 200' eingreift. Weiterhin umfasst das Verriegelungselement 113' wie schon das Verriegelungselement 113 eine Fase 123' am Übergang zwischen plattenförmigem Abschnitt 118' und Randbereich 122', um ein Einführen des Steckers 200' in die Steckbuchse 102' zu erleichtern. Weiterhin umfasst das Verriegelungselement 113' zwei Stifte 119' zur Kraftübertragung zwischen dem Verriegelungselement 113' und dem Betätigungselement.

Abschließend wird im Folgenden anhand von Fig. 11 noch eine weitere alternative Ausführungsform der Steckbuchse beschrieben.

Fig. 11 zeigt den Stecker 200 aus Fig. 1 mit einer zur Fig. 1 weiteren alternativen Ausführungsform einer Steckbuchse 102".

Der in Fig. 11 gezeigte Stecker 200 ist identisch zu dem in Fig. 1 gezeigten Stecker und wird daher an dieser Stelle nicht weiter beschrieben. Weiterhin ist auch eine in Fig. 11 gezeigte Steckbuchse 102" weitestgehend identisch zu der in Fig. 1 gezeigten Steckbuchse 102. Der einzige Unterschied zwischen der Steckbuchse 102 und der Steckbuchse 102" besteht daran, dass die Steckbuchse 102" statt der Ringfederkontakte 130A-H Stiftfederkontakte 130A"-130H" umfasst. Die Stiftfederkontakte 130A"-130H" sind so angeordnet, dass diese bei eingeführtem Stecker 200 in negativer x-Richtung auf die Kontaktflächen 220A-H des Steckers 200 drücken. In anderen Ausführungsbeispielen können die Kontaktstifte jedoch auch anders ausgerichtet sein. Weiterhin ist es auch möglich, dass eine Kontaktfläche an der Spitze des Steckers 200 über ein in Einführungsrichtung 110 angeordneten Kontaktstift abgenommen wird.

Im letzten Bild ist die Verwendung der Anordnung 1 in Verbindung mit einer Blutpumpe 1000 gezeigt. Die Blutpumpe 1000 ist dabei über das Verbindungskabel 300, das den Stecker 200 umfasst, elektrisch verbindbar mit der Steckbuchse 102 der Komponente 100.

Zusammenfassend beschriebt diese Offenbarung eine Anordnung (1) umfassend eine Komponente (100) für eine Blutpumpe und einen Stecker (200), wobei die Komponente (100) zur Herstellung einer elektrischen Verbindung zur Blutpumpe eine Steckbuchse (102) zur lösbaren Aufnahme des Steckers (200) aufweist. Der Stecker (200) weist dabei einen an einer Spitze (202) des Steckers (200) angeordneten und in die Steckbuchse (102) einführbaren Kopplungsabschnitt (204) auf. Ferner umfasst die Steckbuchse (102) ein elektrisch isolierendes Buchsengehäuse (104), das einen Gehäuseeingang (106) und einen sich an den Gehäuseeingang (106) anschließenden Gehäuseinnenraum (108) zur Aufnahme des Kopplungsabschnitts (204) aufweist, wobei eine Flucht vom Gehäuseeingang (106) in den Gehäuseinnenraum (108) eine Einführungsrichtung (110) des Steckers (200) definiert. Zudem umfasst die Steckbuchse (102) eine Arretiereinheit (112), die ausgebildet ist, den in die Steckbuchse (102) eingeführten Stecker (200) lösbar zu arretieren, wobei die Arretiereinheit ausgebildet ist, den Stecker (200) im Buchsengehäuse (104) zu arretieren ohne dabei eine Rotation des Steckers (200) im Buchsengehäuse (104) um die Einführungsrichtung (110) einzuschränken.

## Patentansprüche

1. Anordnung (1), umfassend eine Komponente (100) für eine Blutpumpe und einen Stecker (200), wobei die Komponente (100) zur Herstellung einer elektrischen Verbindung zur Blutpumpe eine Steckbuchse (102) zur lösbaren Aufnahme des Stecker (200) aufweist, wobei
der Stecker (200) einen an einer Spitze (202) des Steckers (200) angeordneten und in die Steckbuchse (102) einführbaren Kopplungsabschnitt (204) aufweist, und
die Steckbuchse (102) aufweist:
ein elektrisch isolierendes Buchsengehäuse (104), das einen Gehäuseeingang (106) und einen sich an den Gehäuseeingang (106) anschließenden Gehäuseinnenraum (108) zur Aufnahme des Kopplungsabschnitts (204) aufweist, wobei eine Flucht vom Gehäuseeingang (106) in den Gehäuseinnenraum (108) eine Einführungsrichtung (110) des Steckers (200) definiert, und
eine Arretiereinheit (112), die ausgebildet ist, den in die Steckbuchse (102) eingeführten Stecker (200) lösbar zu arretieren; wobei
die Arretiereinheit ausgebildet ist, den Stecker (200) im Buchsengehäuse (104) zu arretieren ohne dabei eine Rotation des Steckers (200) im Buchsengehäuse (104) um die Einführungsrichtung (110) einzuschränken.

2. Anordnung (1) nach Anspruch 1, wobei
der Kopplungsabschnitt (204) des Steckers (200) einen, vorzugsweise elektrisch isolierten, Arretierabschnitt (206) umfasst, und
die Arretiereinheit (112) ein Verriegelungselement (113) aufweist, wobei das Verriegelungselement (113) zwischen einer arretierenden Lage und einer nicht-arretierenden Lage verschiebbar ist und in der arretierenden Lage teilweise in die Flucht des Gehäuseeingangs (106) hineinragt und ausgebildet ist, bei eingeführtem Stecker (200) in den Arretierabschnitt (206) des Steckers (200) einzugreifen, um einem Herausziehen des Steckers (200) entgegenzuwirken.

3. Anordnung (1) nach Anspruch 2, wobei der Arretierabschnitt (206) ein oder mehrere laterale Vorsprünge (212) aufweist, die den Kopplungsabschnitt (204) in einer Ebene senkrecht zur Einführungsrichtung (110) zumindest teilweise umgeben und so angeordnet sind, dass diese bei eingeführtem Stecker (200) von dem Verriegelungselement (113) in der arretierenden Lage hintergriffen werden.

4. Anordnung (1) nach Anspruch 3 oder 4, wobei der Arretierabschnitt (206) eine bzgl. der Einführungsrichtung (110) des Steckers (200) den Kopplungsabschnitt (204) umlaufende Auflagefläche (211) aufweist, die an der von der Steckerspitze (208) abgewandten Seite des Vorsprungs (212) bzw. der mehreren Vorsprünge angeordnet ist.

5. Anordnung (1) nach einem der Ansprüche 2 bis 4, wobei
das Verriegelungselement (212) einen plattenförmigen Abschnitt (118) aufweist, der quer, vorzugsweise senkrecht, zur Einführungsrichtung (110) angeordnet ist, und einen Riegelrand (122) umfasst, wobei
der Riegelrand (122) zum Eingreifen in den Arretierabschnitt (206) des Steckers (200) einen Abschnitt (121) mit einem kreisbogenförmigen Verlauf aufweist, der in der arretierenden Lage des Verriegelungselements (113) in die Flucht des Gehäuseeingangs (106) hineinragt.

6. Anordnung (1) nach Anspruch 5, wobei der Riegelrand (122) ein Außenrand des plattenförmigen Abschnitts (118) ist.

7. Anordnung (1) nach Anspruch 5, wobei
der plattenförmige Abschnitt (118) ein Durchgangsloch (120) zum Hindurchführen des Steckers (200) umfasst und
der kreisbogenförmige Riegelrand (121) durch einen Rand des Durchgangslochs (118) gebildet wird.

8. Anordnung (1) nach einem der Ansprüche 5 bis 7, wobei
der plattenförmige Abschnitt (118) eine Stirnfläche umfasst, die in Richtung des Gehäuseeingangs (106) ausgerichtet ist und ein Übergang zwischen Stirnfläche und Riegelrand (118) eine Fase (123) aufweist.

9. Anordnung (1) nach einem der Ansprüche 2 bis 8, wobei
die Arretiereinheit (112) eine Rückstelleinheit (114) umfasst, die ausgebildet ist, wenn keine äußere Krafteinwirkung vorliegt, das Verriegelungselement (113) in der arretierenden Lage zu fixieren.

10. Anordnung (1) nach einem der vorhergehenden Ansprüche, wobei
die Komponente (100) ein Betätigungselement (116) aufweist, das mit der Arretiereinheit (112) verbunden ist, und
die Arretiereinheit (112) ausgebildet ist, bei einem Betätigen des Betätigungselementes (116) die Arretierung aufzuheben.

11. Anordnung (1) nach einem der vorhergehenden Ansprüche, wobei im Kopplungsabschnitt (204) und innerhalb des Gehäuseinnenraums (108) jeweils ein oder mehrere elektrische Kontaktelemente (130A-H, 220A-H) zur Herstellung einer elektrischen Verbindung zwischen der Komponente (100) und dem Stecker (200) angeordnet sind.

12. Anordnung (1) nach Anspruch 11, wobei
der Stecker (200) im Kopplungsabschnitt ein Steckerdichtelement (203) umfasst, und
zwischen Gehäuseeingang (106) und den ein oder mehreren elektrischen Kontaktelementen (130A-H) der Steckbuchse (102) ein Steckbuchsendichtelement (103) angeordnet ist, das ausgebildet ist, bei eingeführtem Stecker (200) mit dem Steckerdichtelement (203) zusammenzuwirken, um einem Eindringen von Flüssigkeit und/oder Partikeln in den Gehäuseinnenraum (108) entgegenzuwirken.

13. Anordnung (1) nach einem der vorhergehenden Ansprüche, wobei
der Gehäuseinnenraum (108), um ein Einführen des Steckers (200) in das Buchsengehäuse (104) zu begrenzen, eine Anschlagfläche (124) umfasst, die in Richtung des Gehäuseeingangs (108) ausgerichtet ist, und
der Stecker (200) mindestens einen lateralen Vorsprung (212) umfasst, der eine in Richtung der Spitze (208) des Steckers (200) ausgerichtete Stirnfläche (216) aufweist, die bei eingeführtem Stecker (200) an der Anschlagsfläche (124) anliegt.

14. Anordnung (1) nach Anspruch 13, wobei die Stirnseite (216) des Vorsprungs (212) eine Fase (213) aufweist.

15. Anordnung (1) nach einem der vorhergehenden Ansprüche, umfassend eine Blutpumpe (1000) und ein Verbindungskabel (300), das den Stecker (200) umfasst und mit dem die Blutpumpe (1000) mit der Komponente (100) elektrisch verbindbar ist.
